# EUROPEAN PATENT SPECIFICATION

(11) **EP 2 231 714 B1**
(45) Date of publication and mention of the grant of the patent: **29.08.2012**
(21) Application number: 09702095.2
(22) Date of filing: 14.01.2009
(51) Int. Cl.: C08F 2/46, C08F 8/50, C08F 22/10, C08G 18/81, C08G 18/28, C08G 18/80

(54) **FUNCTIONAL RESIN COMPOSITION FOR REGULATED POLYMERIZATION STRESS**
FUNKTIONALE HARZZUSAMMENSETZUNG FÜR REGULIERTE POLYMERISIERUNGSBELASTUNG
COMPOSITION DE RÉSINE FONCTIONNELLE POUR UNE CONTRAINTE DE POLYMÉRISATION RÉGULÉE

(30) Priority: 15.01.2008 US 11126 P
(43) Date of publication of application: 29.09.2010
(73) Proprietor: DENTSPLY International Inc., York, PA 17405-0872 (US)
(72) Inventor: JIN, Xiaoming, Middletown DE 19709 (US); O'CONNOR, Mike, Easton MD 21601 (US); HAMMESFAHR, Paul, D., Wyoming DE 19934 (US)
(74) Representative: Blodig, Wolfgang
(86) International application number: PCT/US2009/000229
(87) International publication number: WO 2009/091551

(56) References cited:
- WO-A-2007/146239
- US-A1- 2003 166 738

## Description

### Field of the Invention

This invention relates to "new composition of matter", specifically, photopolymerizable compositions for use as, for example, adhesive, cement, composite. In particular, the use of the new composition of matter is suitable for medical and/or dental materials, as well as applications in other fields where reduction of polymerization stress is important for its durability. This is accomplished by incorporating a photopolymerizable and photocleavable resin based on novel arylketone oxime derivative. Through use of the new composition of matter, the radical polymerization kinetic/rate and thus the resultant polymerization stress is effectively regulated. In addition, the said composition is also capable of generating a new reactive species which in turn creates/initiates a new type of reaction or polymerization for even greater performance enhancement.

### Background of the Invention

Highly cross-linked polymers have been widely studied as matrices for composites, foamed structures, structural adhesives, or insulators for electronic packaging. The densely cross-linked structures are the basis of superior mechanical properties such as high modulus, high fracture strength, and solvent resistance. However, these materials can also be irreversibly damaged by the high contraction stress that is a direct result of the network formation process. It is believed that such contraction or polymerization stress originates from polymerization shrinkage in combination with the limited polymer chain mobility. Consequently, internal stress concentration is elevated and the trapped stress is ultimately released, causing microscopic damage in any weak zones, including within the bulk of the cured material, at the interfacial area, and into the surrounding structures. Macroscopically this effect is often observed as debonding, or cracking. This mechanism is observed in current adhesive dental restorations, wherein polymerization stress from restrained shrinkage build up during cure. The amount of stress within the material is highly dependent on the configuration of the restoration. Furthermore, non-homogeneous deformations resulting from stresses created during functional loading can damage the tooth/composite interface leading to loss of adhesion of the material to tooth structure.

Various approaches have been explored in an effort to reduce the overall polymerization stress generation either from modification of the restorative materials, and/ efforts to minimize the direct stress concentration at the restored interface. They include, for example, new resins, new resin polymerization chemistry, new initiators, new fillers, new coupling agents, new curing sources and processes, new bonding agents, and even new restoration procedures. Significant attention has been directed toward new resin matrix development for low polymerization shrinkage and shrinkage stress. For example, various structure and geometric derivatives of (meth)acrylate-based resin systems, non-(meth)acrylates resin systems, and non-radical-based resin system, have been attempted. In addition, for light curable, low shrink dental composites, new filler and filler surface modifications have also been extensively explored. These include such approaches as fillers of varying particle size and size distributions, ranging from nanometer to micrometer, and different shapes, both irregular or spherical. In addition, it may be of various compositions, such as inorganic, organic (pre-polymerized), or combinations of both (hybrid). Although incremental improvements have been achieved using each approach, polymerization stress is still the biggest challenge in cured network systems.

Almost the entire prior art directed at low shrink and low stress are based on the minimization of the shrinkage and stress formation during the pre-gel phase. However, the shrinkage and stress development in cured network system occurs in two different stages: the pre-gel phase and the post-gel phase. Therefore, some approaches to reduce polymerization shrinkage were found ineffective in controlling over all stress development in the post-gel stage. The immobility caused by the increasing cross-link density within the curing system leads to an increasing stress concentration within the matrix. Even worse, the trapped stress eventually is relieved by slow relaxation, which can create additional damage in the cured system. Therefore, the new approach is based on the concept that in the post-gel stage if some of this "closed network" of the cross-linked system can be broken selectively to promote stress relief, then the total stress concentration within the material will be substantially reduced. This is accomplished using a photopolymerizable and photocleavable resin of a general molecular composition described herein. Such a monomer system described in this work can be polymerized like any other system with the exception that the developed cross-linked network can be "triggered" to break upon the application of an additional energy source, such as additional light energy. The incorporation of the photocleavable monomer within the crosslinked network is believed to be a unique feature of the described new composition of matter.

Photocleavage is utilized in the synthesis of peptides. New peptides are created from an existing template through cleavage of the templates via light exposure. There is no chemical contamination with such a process. As another example, photolytically generated acids and bases can also be viewed as an extended application of photocleavage reactions. In these systems, acidic or basic components are temporally latent to avoid unwanted interaction with other components in the system; they are release on demand such as through light exposure to trigger the generation of the acid or base, which then acts as normal acidic or basic catalysts for secondary reactions. Recently, thermally or photo-chemically reversible materials have been developed in order to make the polymer network depolymerizable or degradable for applications such as easy removal of fill-in polymer in MEMS, thermally labile adhesives, thermaspray coatings and removable encapsulation etc. Most recently, photocleavable dendrimers have been explored in order to improve efficiency of drug delivery.

Previous work disclosed a new approach, which involved a new resin composition that underwent partial selective network photocleavage in the post-gel stage resulting in reduction of polymerization stress. However, the photocleavage process required a relatively low UV wavelength (300nm or less), which is impractical for oral applications due to possible harmful effects to surrounding tissues. Nevertheless, a remarkable low polymerization stress was demonstrated from the curable compositions derived from such a photoactive resin.

It is still highly desirable to further reduce polymerization stress within a cured composite via an effective photocleavage method. Therefore, a new resin composition was explored consisting of a photopolymerizable and photocleavable moiety based on oxime derivatives, which can be cleaved more easily compared to the previous systems described in the prior patent application.

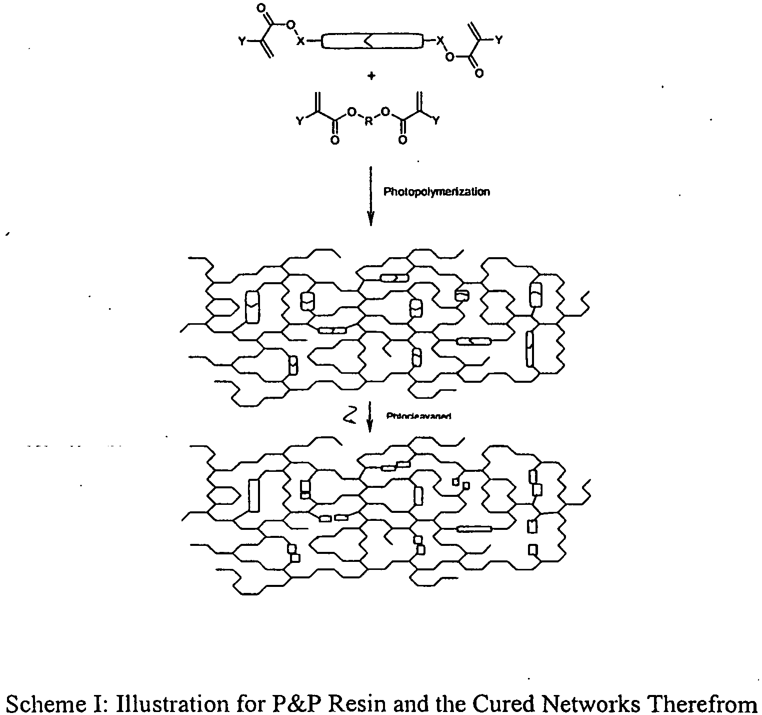

Theoretically, if any environmentally sensitive moiety, such as a thermally cleavable or photo-labile linkage, were incorporated into polymerizable resin monomers, the resulting polymeric material would become thermally cleavable or photo-cleavable. The chemistry of some classical photo-initiators, which have been explored as polymerizable photoinitiator or macroinitiators could be adopted as basis for designing such photopolymerizable and photocleavable resin monomers. However, none of these photo-initiators has been incorporated into a polymer chain or polymeric network to render the polymeric chain or network breakable. Therefore one objective of this invention is to disclose an approach from which a cleavable polymer network is capable of being built up.

It is another objective of this investigation to develop a new resin system for the next generation low shrink and low stress restorative materials by incorporating a photocleavable or thermally liable moiety as part of a photopolymerizable resin monomer. It is expected that such an unusual approach would enable a conventional polymerized network to be selectively cleaved, thus dispersing stress post-polymerization, resulting in a "self" stress-relief system and ultimately minimizing the overall stress concentration within the polymer network.

In order to make a polymerized network cleavable-on-command by light (photocleavable), a light responsive moiety must be incorporated into the polymer backbone that should be stable towards nonnal ambient visible light exposure processes until additional exposure to light with specific energy. In particular, such an energy source can be comprised of other wavelength light other than the standard visible blue light (400mn to 500nm). Near UV light (300nm to 400nm) would be among the many possible choices. Furthermore, it is expected that compounds derived from *ortho*nitrobenzyl radicals or from α-hydroxyalkylphenone should be ideal candidates for this new class of resin monomers that can be photopolymerized by visible light and then triggered to be breakable by additional UV light.

The prior art disclosed in our previous US Patent Application (May, 2004/ June, 2006) described a photopolymerizable and photocleavable resin composition. This approach involves designing a new resin composition that partially relieves the polymerization stress in post-gel stage via a subsequent, selective network cleavage. With this approach, the classic UV photoinitiator, Irgacure 2959, or 4-(2-hydroxyl-ethoxy) phenyl-2- (hydroxy-2-propyl) ketone (HP) was used as the core element in developing a photopolymerizable & photocleavable resin. This resin was used to formulate various dental compositions featuring remarkably low polymerization stress. However, the energy needed to promote the photocleavage falls in the 300nm (UV) range. Although incorporation of an additional photosensitizer was effective at shifting the wavelength to a more desirable range, there remains a need to create a modified photocleavable resin that would undergo photocleavage without the need for a separate, additional photosensitizer.

The prior art,US2005/0182148 (Aug. 18, 2005), disclosed a photosensitive composition in which an ortho-nitrobenzyl moiety was incorporated for photocleavage. However, it did not address any property related polymerization stress reduction or other any reactive species generated from the cleavage process. 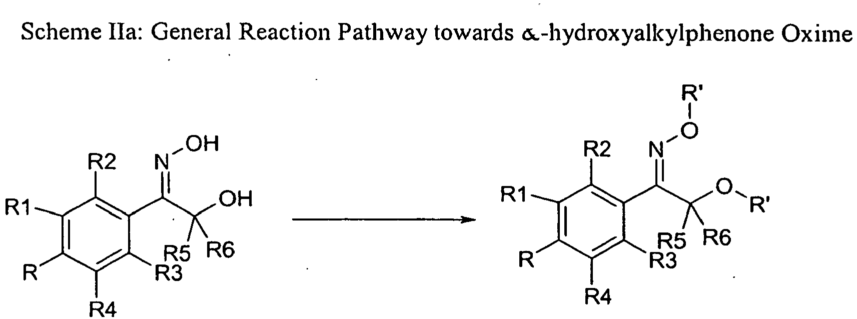
R,R': a linear or branched C1-C18 alkyl or -O-C1-C18 alkyl residue, which can be interrupted by one or more O atoms, PG-Y-R"-X- or a substituted or unsubstituted, aromatic C6 to C18 radiacl;PG; polymerizable group;
R": is absent or a linear or branched C1 to C18 alkylene radical, which can be interrupted by one or more O atoms;
R1, R2, R3, R4: H, a linear or branched C1-C18 alkyl or -O-C1-C18 alkyl residue, which can be interrupted by one or more O atoms, PG-Y-R'-X- or a substituted or unsubstituted, aromatic C6 to C18 radical;
X is absent, O or S; Y is absent, O, S an ester, ether, carbonate, amide or urethane group;
R5 or R6 is H, a line or branched C1-C18 alkyl or -O-C1-C18 alkyl residue, which can be interrupted by one or more O atoms, PG-Y-R'-X- or a substituted or unsubstituted, aromatic C6 to C18 radical;

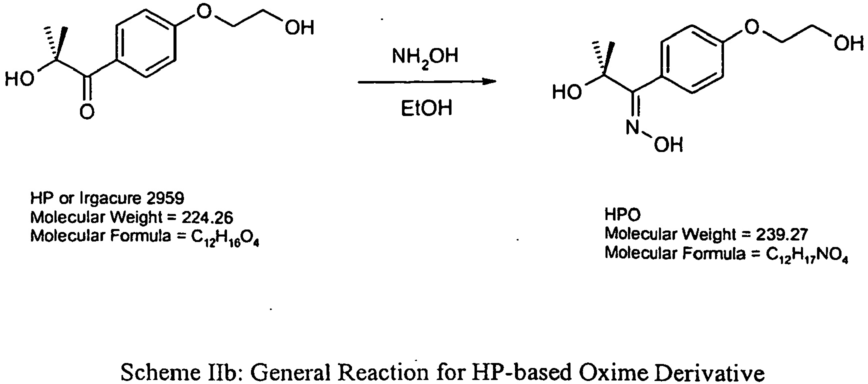

Therefore, there is a desire to further refine the resin composition in order to improve the photocleavability and to better balance the overall mechanical performance along with total polymerization stress reduction. In this invention, a new photoreactive core element, arylketone oxime, was developed. A typical oxime derivative, 4-(2-hydroxylethoxy) phenyl-2-(hydroxy-2-propyl) ketone oxime (HPO) was prepared from its parent ketone compound, 4-(2-hydroxylethoxy)phenyl-2-(hydroxy-2-propyl)ketone (HP) via a facile reaction with hydroxyl amine under mild condition as illustrated in Scheme IIb.

A general resin composition based on such an oxime derivative is further illustrated in Scheme III. As one skilled in the art will recognize, the feasibility of this approach allows for a rapid exploration of a new class of resin monomers. Accordingly, a variety of polymerizable and photocleavable resin monomers has been successfully prepared with a wide range of compositions, as shown in Scheme IV, V and VI.

When di-isocyanate was reacted with HPO, different P&P resin based on HPO resulted due to the varying reactivity of the three different hydroxyl groups within HPO. Further, as illustrated below (Scheme IV, Scheme V and Scheme VI), it was found that hyperbranched P&P resin and non-hyperbranched P&P resin could be effectively prepared via the proper reaction sequence.

Furthermore, different photoreactivity was demonstrated by the P&P resins prepared using slightly different reaction sequence. It appears that the non-hyperbranched P&P resins as shown in Scheme V and VI were more active towards the full spectrum of light than the hyperbranched version as showed in Scheme IV, evidenced by the relatively slow polymerization rate and overall lower polymerization stress that resulted. This can be attributed to the increased photocleavability of the "naked" α-hydroxyl group in non-hyperbranched P&P resin.

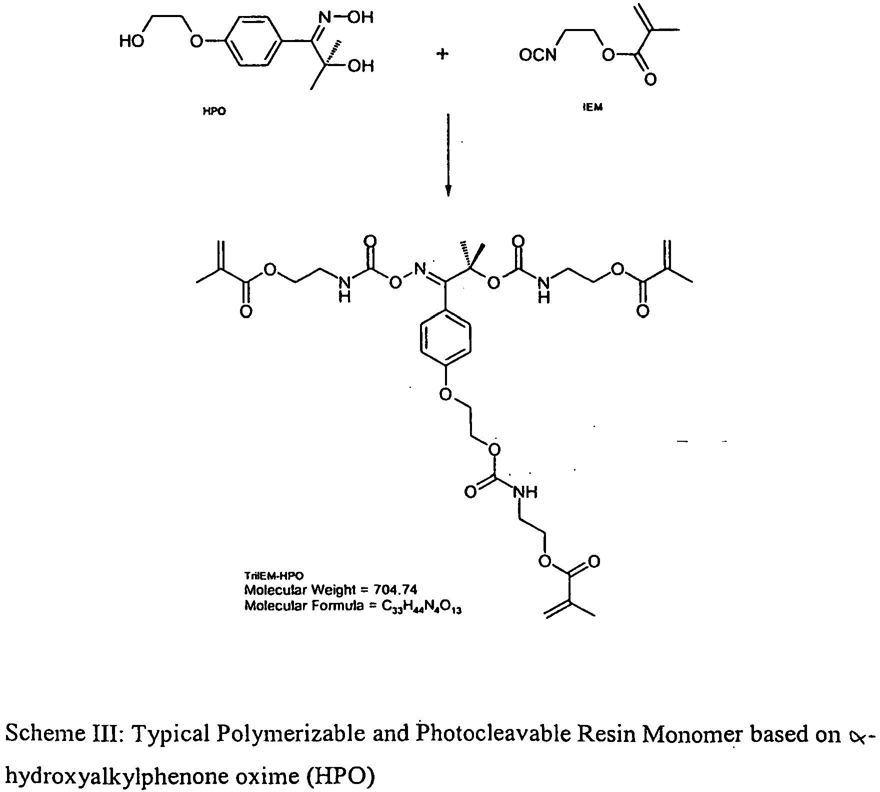

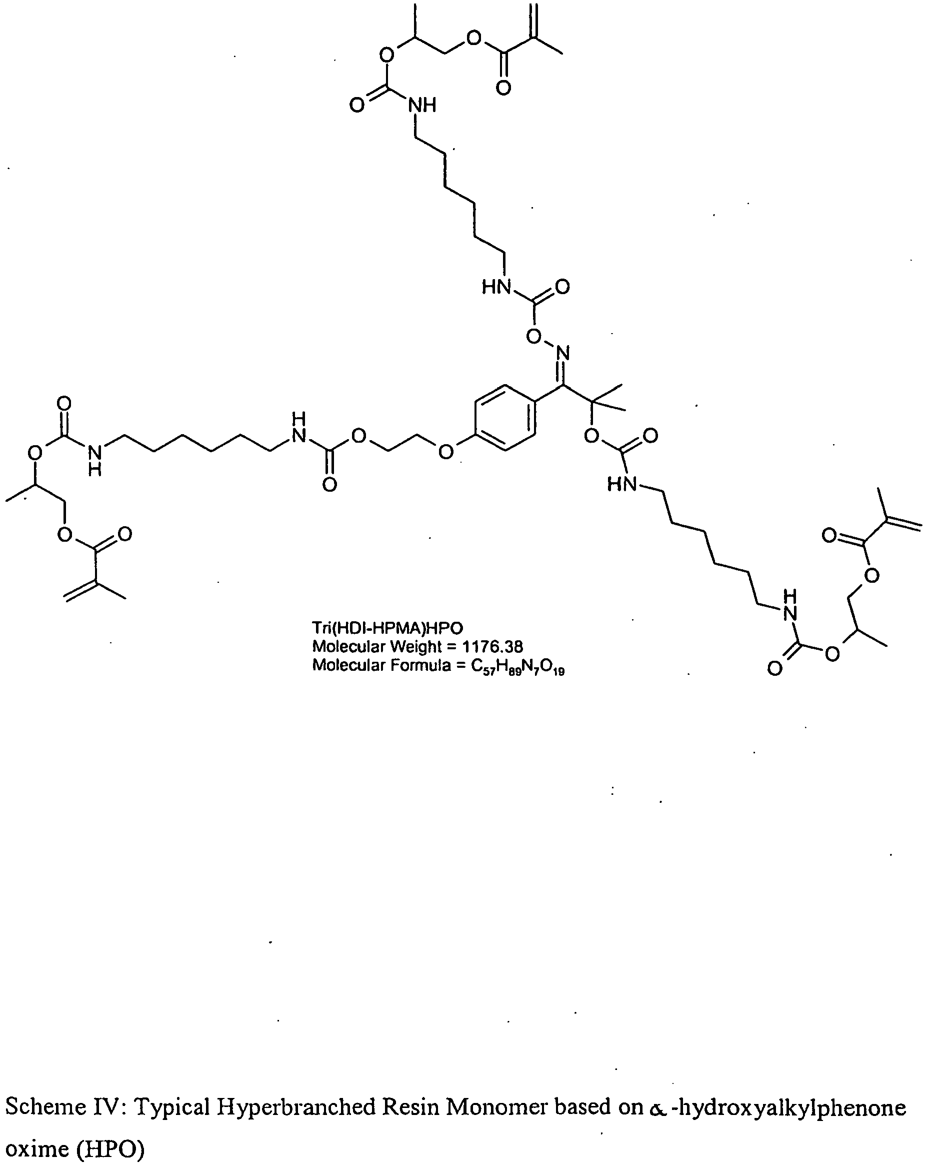

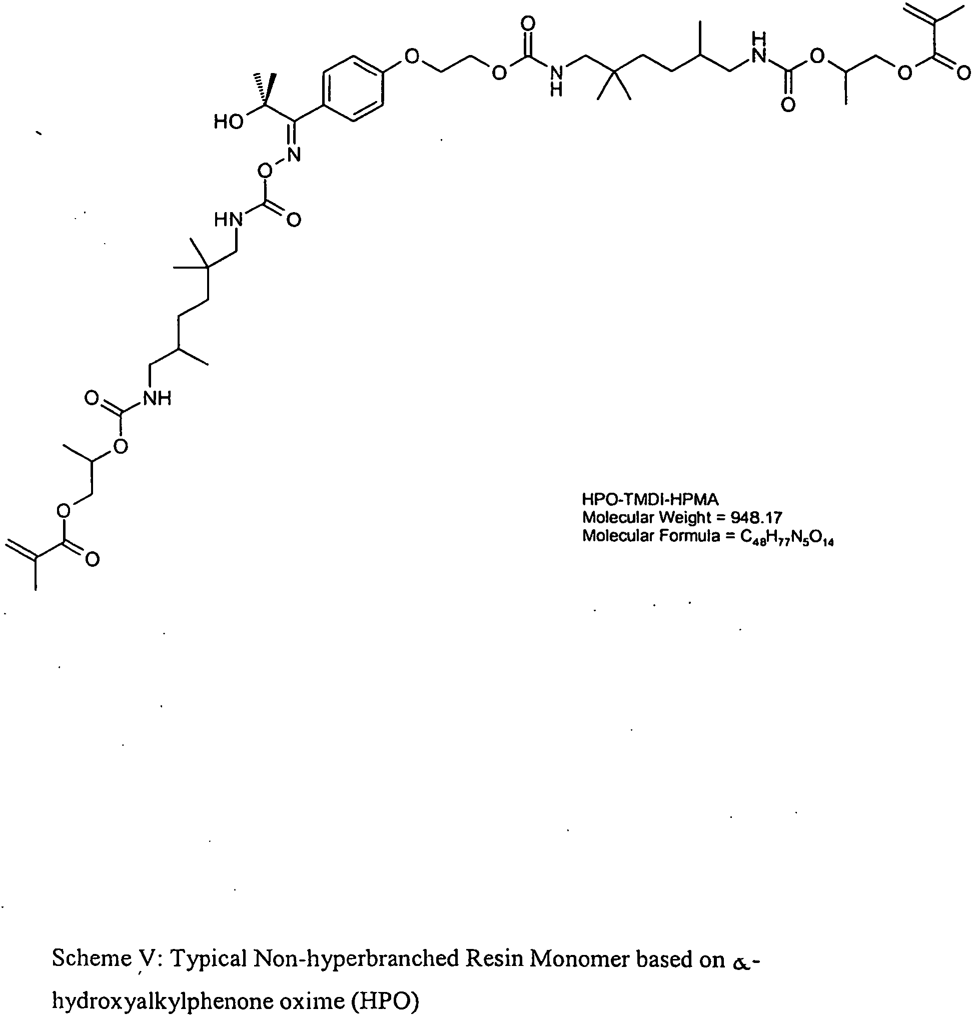

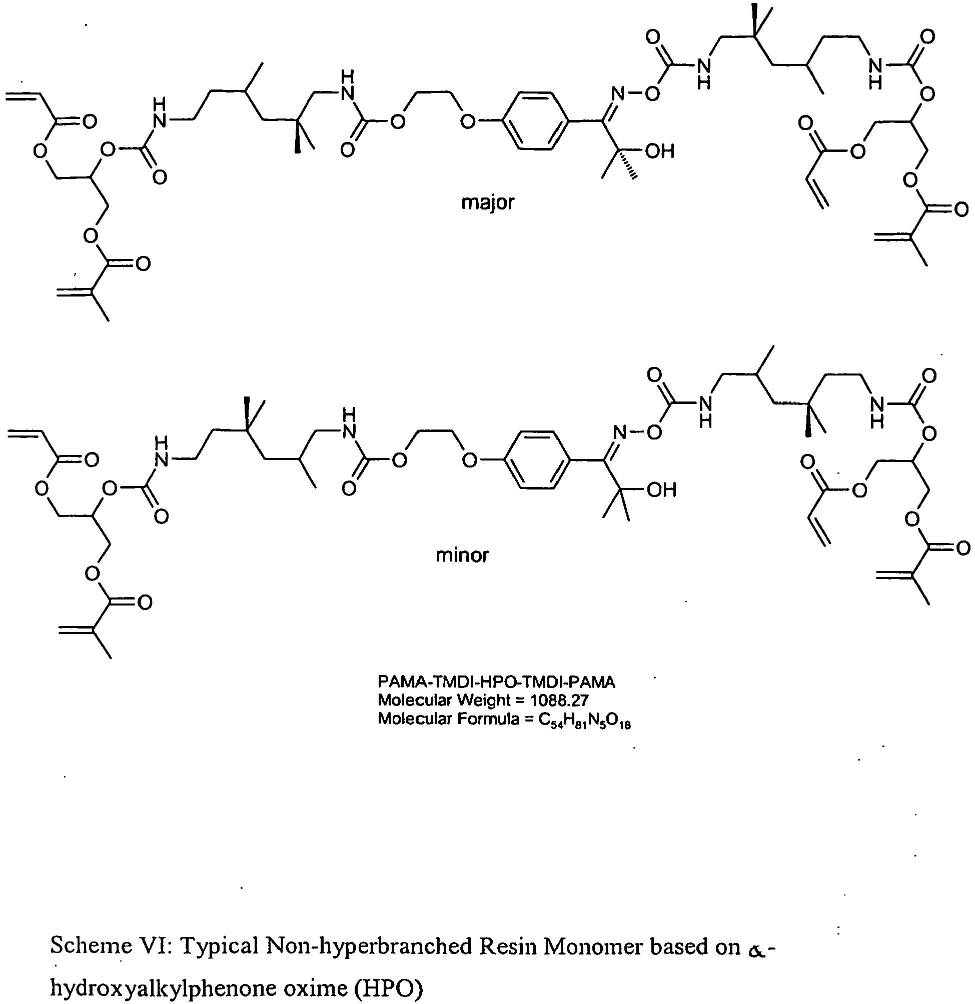

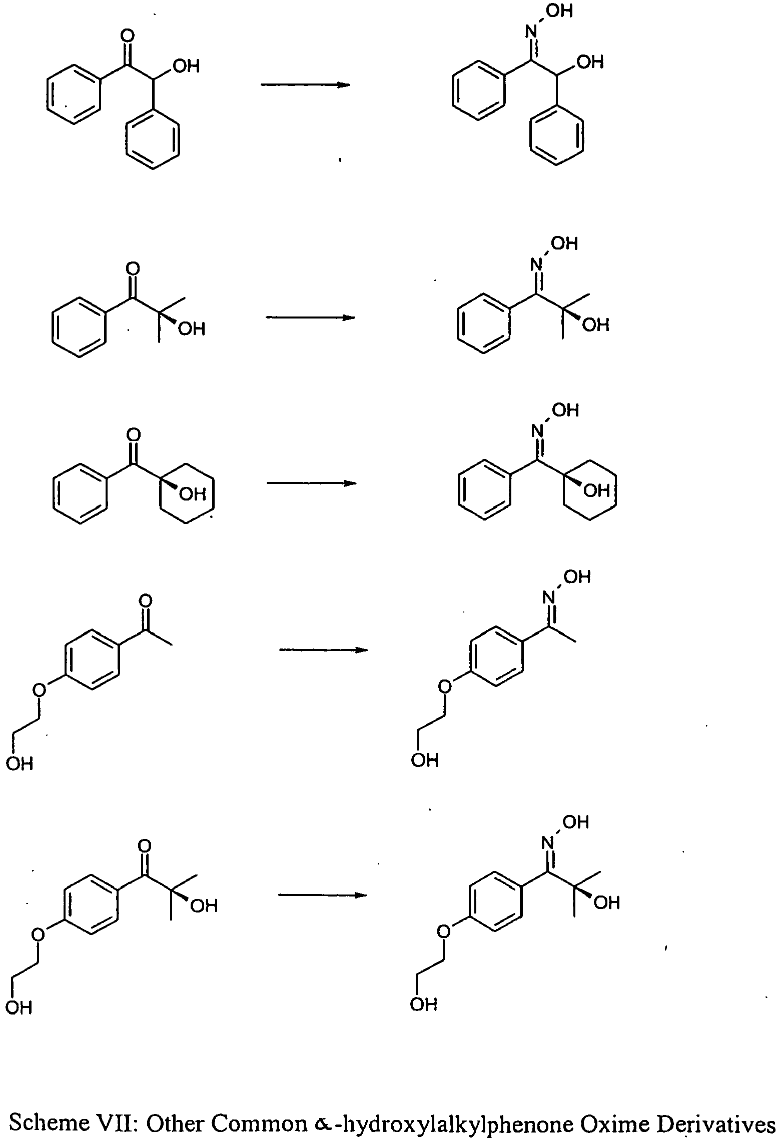

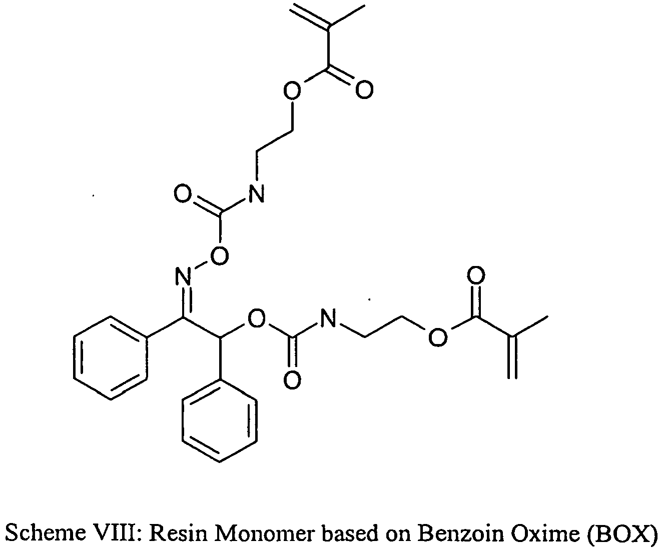

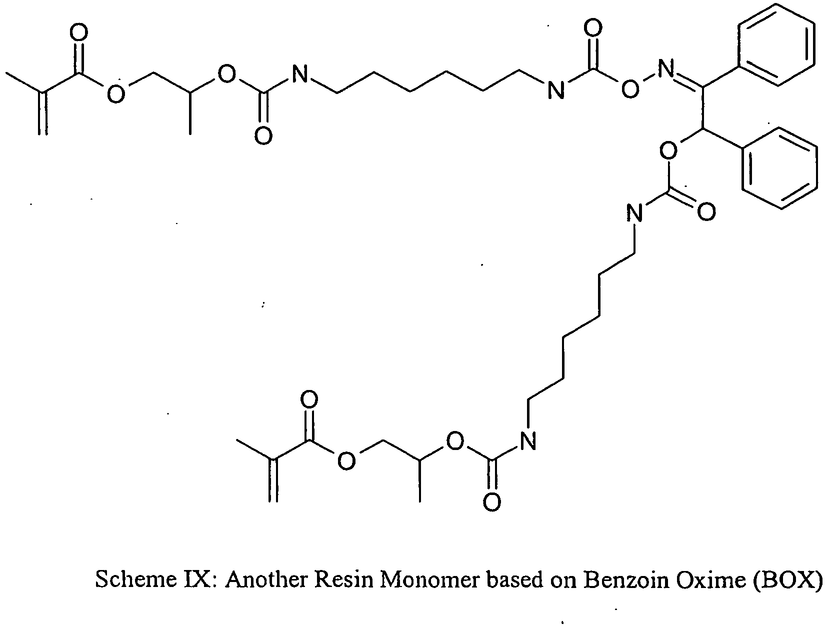

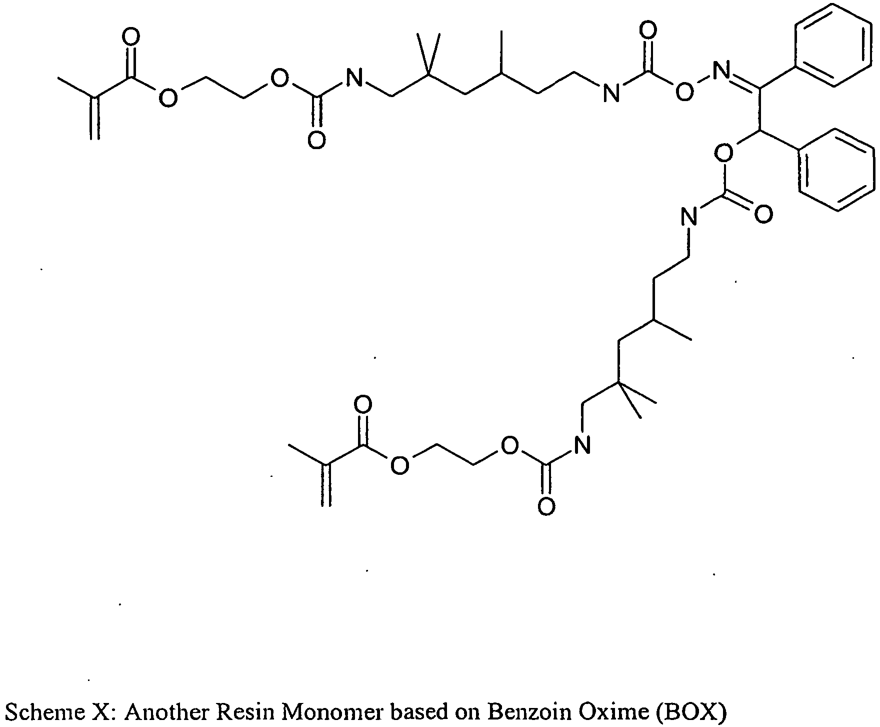

Another important aspect in this invention is that in addition to the active ingredient (P&P resin) that is required to form a photocleavable network after conventional photopolymerization, other photopolymerizable resins should be blended into the composition to aid in establishing a permanent network. A typical activated resin blend contains about 10-90%, w/w, of the P&P resin and 90-10%, w/w, of conventional resins, including in-situ generated UDMA and/or extra diluent resin such as TEGDMA. In addition, some non-radically polymerizable resins, such as epoxy resin, can also be blended with the new P&P resin as a latent reactive diluent which can undergo a typical amine/epoxy addition reaction as a result of photocleavage or *in situ* amine generation. Thus, the addition or blending of additional monomer helps to establish an overall balanced performance of the resulting product, which would include proper viscosity, lower polymerization shrinkage and lower polymerization stress while maintaining good mechanical strength.

A typical composites can be formulated with 10-40%(w/w) of such an activated resin blend along with 60%-90%(w/w) of a filler material composed of a wide range of fillers having different sizes and/or size distributions in order to balance the paste's handling, consistency, and mechanical properties in the resulting composite. A variety of fillers can be used for making the filler blends, including fumed silica, nano filler, polymeric powders, inorganic filers, prepolymerized filler, hybrid filler, et al. For example, the filler blend may be composed as follows: 5-20%(*w*/*w*) silanated BAFG (6-9micron) *5-20%(wlw);* silanated BAFG (0.6-0.8micron) *30-60%(w*/*w)* and Aerosil OX-50 fumed silica (0.01-0.04micron) *5-15%(w*/*w).* Preferably up to *80-85%* (*w*/*w*) of total filler blend can be loaded into the resin matrix.

The novel photocleavable and photopolymerizable resin exhibited a relatively slow polymerization rate, which allows lower polymerization stress buildup even without the photocleavage. In addition, this HPO-based P&P resin demonstrated easier photocleavability than the HP-based P&P resin. Unexpectedly, a further advantage of the systems described herein is the *in situ* amine generation, which triggered subsequently amine-assisted reactions, such as addition with epoxy resin, accelerator for redox initiator, etc.

Furthermore, resin and paste formulations with conventional resin diluent and proper filler composition also produced low shrink and low stress composites characterized by:
- Low polymerization shrinkage(1.0-2.0%, more than 50% lower than conventional composites, 2.8-3.2%);
- Extremely low shrinkage stress (1.0-1.5 MPa, more than 60-70% less than conventional composites, 2.5-3.0MPa);
- An excellent balance of mechanical strength and handling/delivery properties;
- A methacrylate chemistry that makes it compatible with most current bonding systems.

In addition, other physical properties demonstrated by these novel P&P resins resulted in a wide range of physical and mechanical properties that can not be regulated by standard P&P system. These include, but are not necessarily limited to:
- Heat, light, or acidic cleavage
- *in situ* amine generation, which would promote hybrid polymerization in addition to the radical polymerization and make it possible to formulate one-component, dual-cure compositions due to this unique capability to generate a reducing agent, amine, for redox reaction with peroxide.
- Preconditioning to make possible tuning of composite viscosity for better handling and better adaptation to the cavity contours.

Normally, there is an offsetting effect from polymerization stress and mechanical strength whereby enhancing mechanical strength is usually accompanied by increasing polymerization stress due to increasing cross-linking density. It is one purpose of this invention to provide a way to better balance polymerization stress and mechanical properties. Photocleavage during photopolymerization allows a pathway to rearrange covalent bonds. This is particularly significant in a gelling network where larger scale molecular mobility becomes severely restricted.

In the prior art, α-hydroxyalkylphenone is 4-(2-hydroxyethoxy)-phenyl-2-hydroxy-2-methyl-2-propanone, HP, was incorporated with methacylate, acrylate, on vinyl ether, via a one or two-step reaction. Various photopolymerizable HP derivatives have been prepared via ester-ester, ester-carbonate, carbonate-carbonate, and urethane-urethane linkages. Among these, the urethane-urethane linkage offers the most robust process: easy reaction control in a non-solvent process. In addition, the new resin monomers were formulated with other conventional resin monomers such as BisGMA, TEGDMA, UDMA or experimental resin monomers of a macrocyclic nature in a variety of ratios to maximize the overall performance in the resulting composites. As shown in the following examples, remarkably low shrinkage, low stress and excellent mechanical properties, plus good handling characteristics, were demonstrated by those composites based on this new class of P&P resin monomers.

HP must be modified in order to further improve its photoreactivity for an effective photocleavage as discussed above. Here a facile method as described in Example I and Scheme 1, for such a modification from HP to HPO was developed. In addition, as shown in the example a series of P&P resins based on HPO monomer is easily prepared using essentially the same process for HP-based P&P resin. Depending upon the nature of the isocyanate, a one or two-step reactions was utilized to prepare the new P&P resin. IEM is the simplest polymerizable isocyanate. Unfortunately, its toxicity limits its application in biomedical materials. Thus, a simple two-step process was successfully developed to make a phopolymerizable and photocleavable resin: in step I, HPO is capped with diisocyanate to form new diisocyanate in a well-controlled sequence, which was then further reacted with any (meth) acrylate containing a hydroxyl group as shown in step II reaction. This procedure not only allows performing the reaction in one reactor, but also avoids IEM.

Suitable diisocyanates include the alkylene diisocyanates wherein the alkylene group ranges from 2 to about 18 carbon atoms and arylene and substituted arylene di-and polyisocyanates. Thus, exemplary diisocyanates and polyisocyanates include:

Alkaline diisocyanates: ethylene diisocyanate; propylene diisocyanate; tetramethylene diisocyanate; pentamethylene diisocyanate; hexamethylene diisocyanate(HDI); hexamethylene diisocyanate biuret; hexamethylene diisocyanate trimer(isocyanurate); octamethylene diisocyanate; decamethylene diisocyanate; undecamethylene diisocyanate; dodecamethylene diisocyanate; isophorone diisocyanate (IPDI); hydrogenated diphenyl ; methane diisocyanate (H₁₂MDI)

Arylene diisocyanate: xylylene-1,4-diisocyanate(p-XDI); xylylene-1,3-diisocyanate(m-XDI); m-pheylene diisocyanate; p-pheylene diisocyanate; toluene-2,6-diisocyanate(2,6-TDI); toluene-2,4-diisocyanate(2,4-TDI); mesitylene diisocyanate; durylene diisocyanate; benzidene diisocyanate; 1-methyl phenylene-2,4-diisocyanate; naphthylene-1,4-diisocyanate; 1,2,4-benzene triisocyanate; 4,4'-diisocyanato diphenyl methane(MDI); 3,3'-dimethyl-4,4'-diisocyanato diphenyl methane; 4,4'-diphenyl propane diisocyanate; dianisidine diisocyanate; m-tetramethylenexylene diisocyanate(TMXDI)

In addition, suitable (meth)acrylate containing a hydroxyl group include: 2-hydroxyethyl (meth)acrylate; 2-hydroxypropyl (meth)acrylate; 3-(acryloxyl)-2-hydroxypropyl (meth)acrylate; diethylene glycol monohydroxyl (meth)acrylate; triethylene glycol monohydroxyl (meth)acrylate; tetraethylene glycol monohydroxyl (meth)acrylate; polyethylene glycol monohydroxyl (meth)acrylate

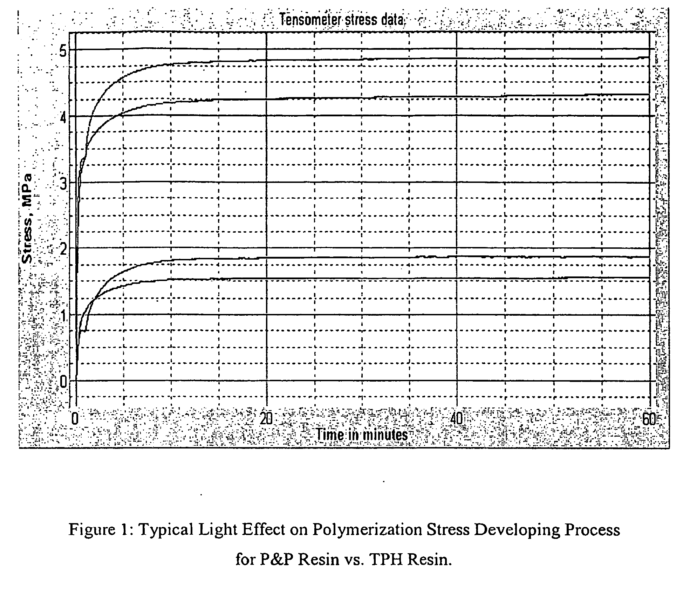

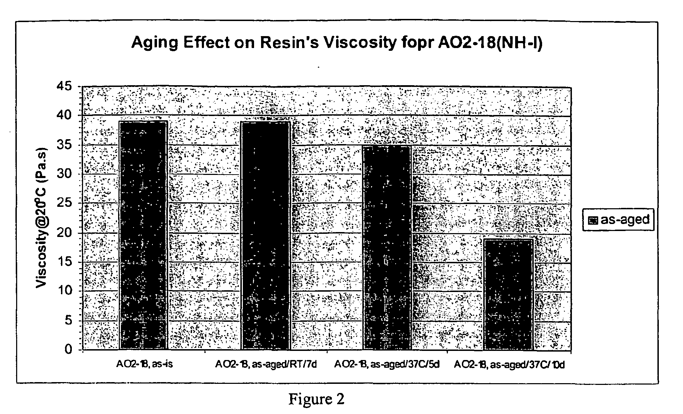

Figure 1 shows polymerization stress as measured by ADA/NIST Tensometer and cured by standard QHT blue light or white light (the UV filter was removed for expanded spectrum and intensity). The novel P&P resins based on HPO, regardless of its structures, hyperbranched or non-hyperbranched type I or II, all exhibit slower curing kinetics which lead to reduced polymerization stress when compared to a conventional resin such as TPH resin. When the curing polymerization is broken into two parts, the stress developed **during light irradiation** (I) and the stress developed in **post light irradiation** (PI), slightly lower percentage of total stress is developed within the novel P&P resin system during part I when it is cured by **blue** light as compared to conventional resin systems such as TPH resin (e.g. 70% vs. 80%). However, when it is cured by **white** light (with UV light included, Table I), significantly different percentages of total stresses developed during and post light irradiation for the novel P&P resin vs. conventional resin. Thus, only 40-60% of total stress was developed using the P&P resin, whereas more than 70% of total stress was developed for TPH resin systems. Such dramatically different response towards **blue** and **white** light for the P&P resin and TPH resin confirms that the P&P resin does undergo special photosensitivity and it also explains the low polymerization stress under blue light curing as a synergetic effect from the same photo-responsive nature but presented in a different magnitude.

It was surprisingly discovered that type I non-hyperbranched P&P resin based on HPO, depending upon its composition as well (e.g. HPMA-HDI-HPO-HDI-HPMA), is more readily degradable at room temperature (a kind of thermal cleavage) as evidenced by the dramatic decrease in viscosity, which was confirmed by NMR analysis. It was also found that such a molecular cleavage would not only lead to a significant change in the composite's consistency or rheological character, but also affected the polymerization rates and overall physical and mechanical properties as well. These may be attributed to the overwhelming presence of free amine that was generated *in situ* during the thermal cleavage process.

On the other hand, it was also discovered that some non-radically polymerizable resins, such as epoxy resin, can become polymerized in the presence of this P&P resin. This was evidenced by the significant mechanical property enhancement for the aged composite system as showed in Table III. The polymerization of the epoxy resin is attributed to the amine generated during cleavage as discussed above. Furthermore, no similar mechanical property enhancement could be found from those composite systems based on any other conventional resin/epoxy formulation because no amine is generated in that case.

Another finding from this cleavable resin system is that an aged P&P resin can be polymerized by addition of BPO alone, even though the aged P&P resin can not be polymerized by any type of light irradiation. This suggests that a one-component, dual-cure system is possible from this invention.

The present invention will now be described in detail with reference to the following examples that do not limit the scope of the invention.

**Table I: Resin Composition based on P&P Resins and TPH Resins**

| Resin Samples | P&P Resin | TEGDM A | ECEHC (Epoxy) | Photoinitiators | TPH Resin | TEGDM A | ECEHC (Epoxy) | Photoinitiators |
|---|---|---|---|---|---|---|---|---|
| | % | % | % | % | % | % | % | % |
| AO1 204 | | | | | **100** | | | CQ/ **0.15** EDAB/ 0.20 |
| AO1-205 | | | | | **90** | **10** | | CQ/ **0.15** EDAB/ **0.20** |
| AO1-1206 | | | | | **90** | | **10** | CQ/ **0.15** EDAB/ **0.20** |
| AO1-207 | | | | | **90** | | **10** | CQ/ **0.15** EDAB/ **0.20** UVI6979/ **1.0** |
| AO1-79 | **85** | **15** | | CQ/0.15 EDAB/ 0.20 | | | | |
| AO1-80 | **80** | **20** | | CQ/ 0.15 EDAB/ 0.20 | | | | |
| AO1-81 | **80** | | **20** | CQ/0.15 EDAB/ 0.20 | | | | |
| AO1-82 | **80** | | **20** | CQ/ 0.15 EDAB/ 0.20 UVI6979/ 0.3 | | | | |
| AO1-101 | **100** | | | CQ/0.15 EDAB/ 0.20 | | | | |
| AO1-102 | **95** | **5** | | CQ/ 0.15 EDAB/ 0.20 | | | | |
| AO1-103 | **80** | | **10** | CQ/0.15 EDAB/ 0.20 UVI6979/ 0.3 | | | | |
| AO2-56 | **100** | | | CQ/ 0.15 EDAB/ 0.20 | | | | |
| AO2-57 | **95** | **5** | | CQ/0.15 EDAB/ 0.20 | | | | |
| AO2-58 | **90** | | **10** | CQ/ 0.15 EDAB/ 0.20 | | | | |
| AO2-59 | **90** | | **10** | CQ/ 0.15 EDAB/ 0.20 UVI6979/ 0.3 | | | | |

**Table II: Light Curing Effect on Featured Polymerization Stress Developing Kinetics: P&P Resins vs. TPH Resins**

| | P&P Resins | TPH Resins | P&P Resins | TPH Resins |
|---|---|---|---|---|
| | PZN Stress During **Blue Light** Irradiation % | PZN Stress During **Blue Light** Irradiation % | PZN Stress During **White Light** Irradiation % | PZN Stress During **White Light** Irradiation % |
| TPH Resin/999446 | | **78** | | **69** |
| AO1-204, as-is | | **78** | | **73** |
| -AO1-205, as-is | | **79** | | **68** |
| AO1-204, as-aged | | **78** | | **73** |
| AO1-205, as-aged | | **79** | | **68** |
| AO1-206, as-is | | **82** | | **70** |
| AO1-207, as-is | | **83** | | **75** |
| AO1-155 | **70** | | **58** | |
| AO1-156 | **70** | | **56** | |
| AO1-157 | **72** | | **64** | |
| AO1-158 | **72** | | **58** | |
| AO1-159 | **56** | | **33** | |
| XJ6-59 | **64** | | **54** | |
| XJ6-60 | **56** | | **29** | |
| AO1-149, as-is | **75** | | **57** | |
| AO1-150, as-is | **74** | | **55** | |
| AO1-151, as-is | **75** | | **60** | |
| AO1-150, as-aged/RT/4d | **71** | | **55** | |
| AO1-101, as-is | **68** | | **42** | |
| AO1-102, as-is | **74** | | **47** | |
| AO1-103, as-is | **70** | | **38** | |
| AO1-124, as-is | **76** | | **57** | |
| AO1-125, as-is | **72** | | **60** | |
| AO1-126, as-is | **75** | | **58** | |
| AO1-144, as-is | **75** | | **58** | |
| AO1-145, as-is | **75** | | **38** | |
| AO1-79, as-is | **61** | | **47** | |
| AO1-80, as-is | **68** | | **36** | |
| AO1-81, as-is | **70** | | **34** | |
| AO1-82, as-is | **68** | | **31** | |
| AO1-40, as-is/aged | **72** | | **44** | |
| AO1-41, as-is/aged | **73** | | **49** | |
| AO1-42, as-is/aged | **70** | | **39** | |
| AO1-43, as-is/aged | **73** | | **41** | |
| AO1-26, as-is/aged | **69** | | **44** | |
| AO1-27, as-is/aged | **73** | | **47** | |
| AO1-28, as-is | **71** | | **49** | |
| AO1-29, as-is | **69** | | **51** | |
| A02-18, as-is | **68** | | **39** | |
| AO2-18, as-aged/RT/7d | **74** | | **38** | |
| AO2-18, as-"aged/37C/5d | **68** | | **35** | |

**Table III: Aging Effect on Mechanical Property for Composites: P&P Resins vs. TPH Resins**

| Composite Samples | Resins | Filler | | | As-is | As-is | As-aged RT/4wks | As-aged RT/4wks |
|---|---|---|---|---|---|---|---|---|
| | | | Polymerizati on Stress | Polymerizat ion Shrinkage | Flexural Strength | Flexural Modulus | Flexural Strength | Flexural Modulus |
| | | % | MPa | % | MPa | MPa | MPa | MPa |
| | | | **2.63** | **3.14** | **106** | **7500** | **107** | **8400** |
| AO2-2 | AO1-204 | **82** | | | | | | |
| | | | **3.15** | **3.22** | **106** | **7760** | **87** | **7400** |
| AO2-3 | AO1-205 | **82** | | | | | | |
| | | | **2.84** | **1.73** | **95** | **6400** | **81** | **6160** |
| AO2-4 | AO1-206 | **82** | | | | | | |
| | | | **3.05** | **1.73** | **89** | **6630** | **81** | **6700** |
| AO2-5 | AO1-207 | **82** | | | | | | |
| | | | **1.64** | **0.86^{.}** | **94** | **8080** | **82** | **7020** |
| AO1-84 | -AO1-79 | **82** | | | | | | |
| | | | **1.88** | **- 0.15** | **94** | **8070** | **86** | **7380** |
| AO1-85 | AO1-80 | **82** | | | | | | |
| | | | **1.01** | **- 0.21** | **62** | **4430** | **93** | **7540** |
| AO1-86 | AO1-81 | **82** | | | | | | |
| | | | **0.87** | **0.92** | **60** | **3850** | **53** | **2450** |
| AO1-87 | AO1-82 | **82** | | | | | | |
| | | **82** | **1.70** | **1.63** | **94** | **8080** | | |
| AO1-105 | AO1-101 | | | | | | | |
| | | | **1.69** | **1.53** | **94** | **8070** | | |
| AO1-106 | AO1-102 | **82** | | | | | | |
| | | | **1.75** | **1.16** | **94** | **7790** | | |
| AO1-107 | AO1-103 | 82 | | | | | | |
| AO2-60 | AO2-56 | **83** | **1.78** | | | | | |
| AO2-61 | AO2-57 | **83** | **1.66** | | | | | |
| AO2-62 | AO2-58 | **83** | **1.77** | | | | | |
| AO2-63 | AO2-59 | **83** | **1.68** | | | | | |

### Example 1: Preparation of HPO

A 1000ml jacketed, cylinder resin kettle equipped with a dropping funnel, mechanical agitator, dry air inlet and water-cooling condenser, through which 35°C of heated water was circulated during the reaction, was charged 650ml 95% Ethanol, 110.0 grams of Irgacure 3959 (HP) and 60.0 grams of NH₂OH.HCl. After the solution turned clear, 120.0 grams of NaOAc was slowly added in portions into the system. It soon turned and remained cloudy through the reaction process, which lasted 2-3 hrs at 35°C. Then it was filtered while the solution was warmed. Dilute aqueous HCl solution was added into the cooled filtrate. Most of ethanol was removed via Rotavapor. White crystals soon developed from the residue. The crystalline powder was filtered and collected, then air-dried and vacuum dried at 50°C over night. A yield of more than 95% was received. ¹H NMR in DMSO-d₆: □10.45ppm (s, 1H), □7.16-7.22pom and □6.90-6.96 (d, 4H), □3.72-3.78 and □3.98-4.04ppm (t, 4H) and 01.24 (s, 6H). ¹³C NMR in DMSO-d₆: □161.715, 158.566, 130.667,126.399, 113.986,72.267, 70.036, 60.256, 29.212ppm, respectively. Tₘ: 131.6°C (DSC).

### Example 2: Preparation of the adduct of (IEM)₂-HPO-IEM (AO1-16).

A 250ml three-necked flask equipped with a mechanical agitator, dry air inlet and water-cooling condenser was immersed in an oil-bath and charged with 55.8 grams of IEM, 0.15 grams of DBTDL, and 0.188 gram of BHT. Then it was charged in portions with 24.0 grams of HPO over 4hrs. The reaction was kept at 35°C with the oil-bath temperature. The reaction took place for 2hrs before adding 11.5 grams of HPMA. The reaction ran for an additional 20hrs at this temperature. The total yield was 95%.

### Example 3: Preparation of the adduct of (HPMA-HMD)₂-HPO-HMDI-HPMA (AO1-30)

A 500ml three-necked flask equipped with a powder addition funnel, mechanical agitator, dry air inlet and water-cooling condenser was immersed in an oil-bath and charged with 95.2 grams of HMDI and 0.21 gram of DBTDL. Then 30.1 grams of sieved HPO was slowly added in portions to the flask over a period of 3hrs. This slow addition took place in such a manner to avoid a rapid reaction heat increase, which might jeopardize the desired sequence of HMDI-HPO-HMDI. The reaction ran at 35 °C overnight.. Then 0.12gram of BHT was charged into the system. With a continuous purge of dry air into the reaction system, 112.5 grams of HPMA was added to the flask through a dropping funnel over a period of 2hrs. After HEMA addition, the reaction was allowed to proceed for an additional 3-4 hrs at 35 °C. Then 25.0 grams of TEGDMA was added as diluent into system and mixed for approximately 2 hours prior to discharge. The overall yield was about 97%. The resin had a viscosity of 160 Pa.s at 20 °C.

### Example 4: Preparation of the adduct of HEMA-TMDI-HPO-TMDI-HEMA (AO1-76)

A 500ml jacketed, cylinder resin kettle equipped with a powder addition funnel, mechanical agitator, dry air inlet and water-cooling condenser, through which 35 °C of heated water was circulated during the reaction, was charged with 96.8 grams of TMDI, 0.10 gram of BHT, and 0.22gram of DBTDL. 66.0 grams of HPMA was added into the system through a dropping funnel within a period of 2hrs. After an additional 2hrs of reaction at this temperature, 52.3 grams of sieved HPO was fed slowly in portions into the system over a period of 1.5hrs. Finally, 40.0 grams of TEGDMA was added into the system two hours later and then mixed and discharged with a yield of 97%. This resin showed a viscosity of 1600 Pa.s at 20 °C.

### Example 5: Preparation of the adduct of HEMA-TMDI-HPO-TMDI-HEMA (AO1-97)

A 500ml jacketed, cylinder resin kettle equipped with a powder addition funnel, mechanical agitator, dry air inlet and water-cooling condenser, through which 35 °C of heated water was circulated during the reaction, was charged with 96.8 grams of TMDI, 0.10 gram of BHT, and 0.22gram of DBTDL. 66.3 grams of HPMA was added into the system through a dropping funnel over a period of 3hrs. After an additional 1hr of reaction at this temperature, 35.1 grams of sieved HPO was fed slowly in portions into the system over a period of 1hr. Finally, 40.0 grams of TEGDMA was added into the system one hour later and then 24.0 grams of HEMA was added drop-wise into the reaction system over a one hour period. It was mixed overnight at 35°C and discharged with yield of 97%. This resin had a viscosity of 95 Pa.s at 20 °C.

### Example 6: Preparation of the adduct of HPAMA-TMDI-HPO-TMDI-HPAMA (XJ6-53)

A 500ml jacketed, cylinder resin kettle equipped with a powder addition funnel, mechanical agitator, dry air inlet and water-cooling condenser, through which 35 °C heated water was circulated during the reaction, was charged with 96.8 grams of TMDI, 0.10 gram of BHT, and 0.22gram of DBTDL. 99.0 grams of HPAMA was added into the system through a dropping funnel within a period of 6hrs. After additional overnight reaction at this temperature, 40.0 grams of TEGDMA was charged into this system. Then 35.2 grams of sieved HPO was fed slowly in portions into the system over a period of 2hrs. Finally, 24.0 grams of HEMA was added drop-wise into the reaction system over a two-hour period. It was mixed overnight at 35 °C and discharged with yield of 97%. The viscosity was 195 Pa.s at 20 °C.

### Comparative Example 1: Preparation of the adduct of HEMA-TMDI-BOX-TMDI-HEMA (XJ6-64)

A 1000ml jacketed, cylinder resin kettle equipped with a powder addition funnel, mechanical agitator, dry air inlet and water-cooling condenser, through which 35°C of heated water was circulated during the reaction, was charged with 193.5 grams of TMDI, 0.15 gram of BHT, and 0.39gram of DBTDL. 132.6 grams of HPMA was added into the system through a dropping funnel within a period of 8hrs. After additional overnight reaction at this temperature, 68.2 grams of sieved benzoin oxime (BOX) was added slowly in portions into the system over a period of 1.5hrs. The reaction proceeded at this temperature for another three hours before 80 grams of TEGDMA was added into the system, which was then followed by adding 49.2 grams of HEMA for an additional two hours prior to stopping the reaction. 515 grams of clear resin resulted with a yield of 98%. After one week at room temperature, the resin was found partially gelled, and could not be further formulated to make any pastes.

### Resin Formulation Example 1 to 11

Typical activated resin composition examples were formulated as described in Table I. Additional resin compositions were also formulated as listed in Table II, in which different types of P&P resin were formulated with a variety of other polymerizable but non-cleavable resin, such as TEGDMA or other non-radically-polymerizable resin, such as EHECH epoxy resin in presence/absence of its photocationic initiators, UVL6979, see AO1-79, 80, 81, 82, AO1-101, 102, 103, and AO2-56, 57, 58, 59, respectively.

### Comparative Resin Formulation Example 1 to 4

In addition, comparative resin compositions based on a conventional resin, a urethane-modified BisGMA (TPH resin) was also formulated in a way similar to those compositions as described above for P&P resin and listed in Table I as well, AO1-201, 202, 203 and 204.

### Composite Formulation Example 1 to 11

Typical composite composition examples were accordingly further formulated as described in Table III, AO1-84, 85, 86, 87, AO1-105, 106, 107, AO2-60, 61, 62, and 63, respectively. Furthermore, some of these pastes were evaluated after several weeks' aging at room temperature in order to demonstrate the aging-induced nature for those new P&P resins.

### Comparative Composite Formulation Example 1 to 4

In addition, comparative composite examples were accordingly further formulated as described in Table III as well, AO2-2, 3, 4, and 5, respectively. Again, some of these pastes were evaluated after several weeks' aging at room temperature in order to make a direct comparison on the aging effect on these non-cleavable systems to the novel P&P resin-based systems.

## Claims

1. A photopolymerizable and photocleavable resin monomer derived from a reactive photosensitive moiety via various linkages to form photopolymerizble monomers and/or oligomers as described in following formula, which contains at least one polymerizable group within each resin monomer: 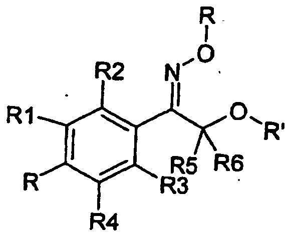
R, R': H, a linear or branched C1-C18 alkyl or -O-C1-C18 alkyl residue, which can be interrupted by one or more O atoms, PG-Y-R"-X- or a substituted or unsubstituted, aromatic C6 to C18 radiacl;PG; polymerizable group;
R": is absent or a linear or branched C1 to C18 alkylene radical, which can be interrupted by one or more O atoms;
R¹, R², R³, R⁴: H, a linear or branched C1-C18 alkyl or -O-C1-C18 alkyl residue, which can be interrupted by one or more O atoms, PG-Y-R'-X- or a substituted or unsubstituted, aromatic C6 to C18 radical;
X is absent, O or S;
Y is absent, O, S an ester, ether, carbonate, amide or urethane groups; R⁵ or R⁶ is H, a linear or branched C1 -C18 alkyl or -O-C1-C18 alkyl residue, which can be interrupted by one or more O atoms, PG-Y-R'-X- or a substituted or unsubstituted, aromatic C6 to C18 radical.

2. The photopolymerizable and photocleavable resin monomer as claimed in 1 , wherein the reactive and photo-reactive moiety is a reactive D-hydroxyalkylphenone oxime, such as 4-(2-hydroxyethoxy)-phenyl-2-hydroxy-2- methyl-2-propanone oxime(HPO) or any other functional photo-reactive compound with oxime moiety.

3. The photopolymerizable and photocleavable resin monomer as claimed in 1 and 2, wherein the photopolymerizable and photocleavable resin monomer is constructed via any linkages such as ester, carbonate, urea, urethane, ether or their combination from the existing reactive end groups, or hydroxyl groups.

4. The photopolymerizable and photocleavable resin monomer as claimed in 1 and 2, wherein the photopolymerizable and photocleavable resin monomer bear at least two photopolymerizable groups.

5. The photopolymerizable and photocleavable resin monomer as claimed in 4, wherein the photopolymerizbale groups are vinyl, vinoylether, acrylate, methacrylate, or their combination.

6. The photopolymerizable and photocleavable resin monomer as claimed in 3, wherein such photopolymerizable and photocleavable (P&P) resins are derivatives of urethane-based monomers obtainable via a one-step or two-step process.

7. The photopolymerizable and photocleavable resin monomer as claimed in 6, wherein diisocyanates including alkylene diisocyanates wherein the alkylene group ranges from 2 to about 18 carbon atoms, and arylene and substituted arylene di-and polyisocyanates are used to make the urethane-based P&P resin

8. Medicinal or dental material containing the photopolymerizable and photocleavable (P&P) resin monomer according to any one of the preceding claims and a conventional resin including BisGMA, TEGDMA, UDMA, HEMA, HPMA.

9. The medicinal or dental material as claimed in claim 8, wherein the content of such photopolymerizable and photocleavable (P&P) resin monomer in other resin mix range from 0.01% to 99.99%, preferably 0.5 to 99.5%, more preferably 1.0 to 60.0%, w/w, respectively.

10. The medicinal or dental material as claimed in 8, wherein the photopolymerizable and photo-reactive system is also activable by a second energy source, separate from the first source used to photopolymerize the first reaction, whereby preferably a small fraction of less than 10% of such resin can be used as functional ingredient to promote secondary reaction or event on command as needed.

11. The medicinal or dental material as claimed in claim 10, wherein such a command can be any external energy such as heat, light with given wavelength, microwave, ultrasonic or mechanical vibration.

12. The medicinal or dental material as claimed in 10 and 11, wherein such a secondary event or reaction including viscosity change, amine regeneration, acid regeneration; or wherein such a composition may be utilized in making viscosity tunable materials; or wherein such a composition may be utilized in one-component, dual-cure materials; or wherein such a composition could be utilized as stable self-etching system.

13. Use of a photopolymerizable and photocleavable resin monomer as claimed in any one of claims 1 to 7 for reducing polymerization stress of a paste composition containing a filler.

14. The use as claimed in claim 13, wherein the resin composition as claimed in 1 and 2 is used in the paste composition from 1 % -99%, wt/wt, preferably wherein the filler is a filler composition containing a variety of size, size distribution including nano filler, micro filler and macro filler or hybrid filler in terms of size and composition or nature, that is inorganic or organic or prepolymerized.

15. The use as claimed in claims 13 and 14, wherein the filler composition is surface treated by coupling agents, polymerizable or non-polymerizable silane or their combination to further improve physical or mechanical property.

## Patentansprüche

1. Photopolymerisierbares und photospaltbares Harzmonomer, abgeleitet von einer reaktiven photoempfindlichen Einheit über verschiedene Bindungen, um photopolymerisierbare Monomere und/oder Oligomere, wie sie durch folgende Formel beschrieben werden, zu bilden, das mindestens eine polymerisierbare Gruppe innerhalb jedes Harzmonomeres enthält: 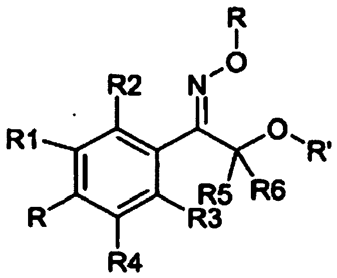
R, R': H, ein lineares oder verzweigtes C1-C18 Alkyl oder -OC1-C18 Alkylrest, der unterbrochen sein kann von einem oder mehreren O-Atomen, PG-Y-R"-X- oder ein substituiertes oder unsubstituiertes aromatisches C6-C18 Radikal; PG; polymerisierbare Gruppe;
R": ist abwesend oder steht für ein lineares oder verzweigtes C1 bis C18 Alkylenradikal, das von einem oder mehreren O-Atomen unterbrochen sein kann;
R¹, R², R³, R⁴: H, ein lineares oder verzweigtes C1-C18 Alkyl oder -O-C1-C18 Alkylrest, der unterbrochen sein kann durch ein oder mehrere O-Atome, PG-Y-R'-X- oder ein substituiertes oder unsubstituiertes aromatische C1-C18 Radikal ;
X fehlt oder für O oder S steht;
Y fehlt oder für O, S, eine Ester-, Ether-, Carbonat-, Amid- oder Urethangruppe steht;
R⁵ oder R⁶ steht für H, ein lineares oder verzweigtes C1-C18 Alkyl oder - O-C1-C18 Alkylrest, der unterbrochen sein kann durch ein oder mehrere O-Atome, PG-Y-R'-X- oder ein substituiertes oder unsubstituiertes aromatisches C6 bis C18 Radikal.

2. Das photopolymerisierbare und photospaltbare Harzmonomer nach Anspruch 1, wobei die reaktive und photoreaktive Einheit ein reaktives D-Hydroxyalkylphenonoxim, wie 4-(2-Hydroxyethoxy)-phenyl-2-hydroxy-2-methyl-2-propanonoxim (HPO) oder irgendeine andere funktionelle photoreaktive Verbindung mit einer Oximeinheit ist.

3. Das photopolymerisierbare und photospaltbare Harzmonomer nach Anspruch 1 und 2, wobei das photopolymerisierbare und photospaltbare Harzmonomer aufgebaut ist über Bindungen, wie Ester-, Carbonat-, Harnstoff-, Urethan-, Etherbindungen oder deren Kombinationen aus den vorhandenen reaktiven Endgruppen, oder Hydroxylgruppen.

4. Das photopolymerisierbare und photospaltbare Harzmonomer nach Anspruch 1 und 2, wobei das photopolymerisierbare und photospaltbare Harzmonomer mindestens zwei photopolymerisierbare Gruppen trägt.

5. Das photopolymerisierbare und photospaltbare Harzmonomer nach Anspruch 4, wobei die photopolymerisierbaren Gruppen Vinyl-, Vinoylether-, Acrylat-, Methacrylat-, oder Kombinationen aus deren Gruppen sind.

6. Das photopolymerisierbare und photospaltbare Harzmonomer nach Anspruch 3, wobei solche photopolymerisierbaren und photospaltbaren (P&P) Harze Derivate eines Urethan-basierten Monomers sind, die erhältlich sind über einen einstufigen oder zweistufigen Prozess.

7. Das photopolymerisierbare und photospaltbare Harzmonomer nach Anspruch 6, wobei Diisocyanate, einschließlich Alkylendiisocyanate, wobei die Alkylengruppe von 2 bis 18 Kohlenstoffatome aufweist, und Arylen und substituierte Arylen Di- und Polyisocyanate verwendet werden, um das Urethan-basierte P&P Harz herzustellen.

8. Medizinisches oder dentales Material, enthaltend das photopolymerisierbare und photospaltbare (P&P) Harzmonomer nach einem der vorstehenden Ansprüche sowie ein herkömmliches Harz, einschließlich BisGMA, TEGDMA, UDMA, HEMA, HPMA.

9. Das medizinische oder dentale Material nach Anspruch 8 wobei der Gehalt solcher photopolymerisierbarer und photospaltbarer (P&P) Harzmonomere in dem Harzgemisch im Bereich von 0,01% bis 99,99%, vorzugsweise 0,5 bis 99,5%, noch bevorzugterweise 1,0 bis 60,0%, Gewicht/Gewicht, jeweils liegt.

10. Das medizinische oder dentale Material nach Anspruch 8, wobei das photopolymerisierbare und photoreaktive System auch aktivierbar ist durch eine zweite Energiequelle, die unabhängig von der ersten Quelle ist, welche verwendet wird, um die erste Reaktion zu photopolymerisieren, wobei vorzugsweise ein kleiner Anteil von weniger als 10% eines solchen Harzes verwendet werden kann als funktioneller Bestandteil, um eine Sekundärreaktion oder -Kleinereignis, je nach Bedarf zu fördern.

11. Das medizinische oder dentale Material nach Anspruch 10, wobei solch ein Kommando irgendeine äußere Energie sein kann, wie Wärme, Licht, das eine bestimmte Wellenlänge aufweist, Mikrowellen, Ultraschall- oder mechanische Vibration.

12. das medizinische oder dentale Material nach Anspruch 10 und 11, wobei ein solches Sekundärereignis oder -Kleinreaktion beinhaltet eine Viskositätsänderung, eine Aminregeneration, eine Säureregeneration; oder wobei eine solche Zusammensetzung verwendet werden kann bei der Herstellung von Viskositäts-einstellbaren Materialien; oder wobei eine solche Zusammensetzung verwendet werden kann in einkomponentigen Materialien, die nach zwei Härtungsmechanismen härten; oder wobei eine solche Zusammensetzung verwendet werden kann als stabiles selbstätzendes System.

13. Verwendung eines photopolymerisierbaren und photospaltbaren Harzmonomers, wie es in irgendeinem der Ansprüche 1 bis 7 beanspruche wird, zur Verminderung einer Polymerisationsspannung einer Pastenzusammensetzung, die einen Füllstoff aufweist.

14. Die Verwendung nach Anspruch 13, wobei die Harzzusammensetzung wie sie in Anspruch 1 und 2 beansprucht wird, in der Pastenzusammensetzung in einer Menge von 1% - 99% Gewicht/Gewicht eingesetzt wird, vorzugsweise wobei der Füllstoff eine Füllstoffzusammensetzung ist, die eine Mehrzahl von Größen, Größenverteilungen aufweist, einschließlich Nanofüllstoffen, Mikrofüllstoffen und Makrofüllstoffen, oder einen Hybridfüllstoff in Bezug auf Größe und Art der Zusammensetzung, die anorganisch oder organisch oder vorpolymerisiert sein kann.

15. Die Verwendung nach Anspruch 13 und 14, wobei die Füllstoffzusammensetzung oberflächenbehandelt ist mit Haftvermittlern, polymerisierbaren oder nichtpolymerisierbaren Silanen oder einer Kombination davon, um physikalische oder mechanische Eigenschaften weiter zu verbessern.

## Revendications

1. Monomère de résine photopolymérisable et photoclivable dérivé d'un groupement photosensible réactif par diverses liaisons pour former des monomères et/ou oligomères photopolymérisables de la manière décrite dans la formule suivante, qui contient au moins un groupe polymérisable dans chaque monomère de résine : 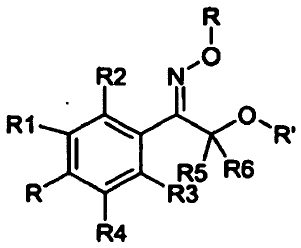
R, R' : H, un groupe alkyle en C₁ à C₁₈ linéaire ou ramifié ou résidu -O-(alkyle en C₁ à C₁₈), qui peut être interrompu par un ou plusieurs atomes de O, un groupe PG-Y-R"-X- ou un radical en C₆ à C₁₈ aromatique substitué ou non substitué ;
PG : groupe polymérisable ;
R" : est absent ou représente un radical alkylène en C₁ à C₁₈ linéaire ou ramifié, qui peut être interrompu par un ou plusieurs atomes de O ;
R¹, R², R³, R⁴ : H, un groupe alkyle en C₁ à C₁₈ linéaire ou ramifié ou résidu -O- (alkyle en C₁ à C₁₈), qui peut être interrompu par un ou plusieurs atomes de O, un groupe PG-Y-R' -X- ou un radical en C₁ à C₁₈ aromatique substitué ou non substitué ;
X est absent ou représente O or S ;
Y est absent ou représente O, S, un groupe ester, éther, carbonate, amide ou uréthanne ;
R⁵ ou R⁶ représente H, un groupe alkyle en C₁ à C₁₈ linéaire ou ramifié ou résidu -O-(alkyle en C₁ à C₁₈), qui peut être interrompu par un ou plusieurs atomes de O, un groupe PG-Y-R'-X- ou un radical en C₆ à C₁₈ aromatique substitué ou non substitué.

2. Monomère de résine photopolymérisable et photoclivable suivant la revendication 1, dans lequel le groupement réactif et photoréactif est une oxime de D-hydroxyalkylphénone réactive, telle que l'oxime de 4-(2-hydroxyéthoxy)-phényl-2-hydroxy-2-méthyl-2-propanone (HPO) ou n'importe quel autre composé photoréactif fonctionnel avec un groupement oxime.

3. Monomère de résine photopolymérisable et photoclivable suivant les revendications 1 et 2, ledit monomère de résine photopolymérisable et photoclivable étant construit par n'importe quelles liaisons telles que des liaisons ester, carbonate, urée, uréthanne, éther ou leur association à partir des groupes terminaux réactifs existant, ou des groupes hydroxyle.

4. Monomère de résine photopolymérisable et photoclivable suivant les revendications 1 et 2, ledit monomère de résine photopolymérisable et photoclivable portant au moins deux groupes photopolymérisables.

5. Monomère de résine photopolymérisable et photoclivable suivant la revendication 4, dans lequel les groupes photopolymérisables sont des groupes vinyle, vinoyléther, acrylate, méthacrylate ou leur association.

6. Monomère de résine photopolymérisable et photoclivable suivant la revendication 3, dans lequel les résines photopolymérisables et photoclivables (P&P) sont des dérivés de monomères à base d'uréthanne pouvant être obtenus par un procédé en une étape ou un procédé en deux étapes.

7. Monomère de résine photopolymérisable et photoclivable suivant la revendication 6, dans lequel des diisocyanates comprenant des alkylène-diisocyanates dans lesquels le groupe alkylène a 2 à environ 18 atomes de carbone, et des arylène- et (arylène substitué)- di- et poly-isocyanates sont utilisés pour préparer la résine P&P à base d'uréthanne.

8. Matière médicale ou dentaire contenant le monomère de résine photopolymérisable et photoclivable (P&P) suivant l'une quelconque des revendications précédentes et une résine classique comprenant les BisGMA, TEGDMA, UDMA, HEMA, HPMA.

9. Matière médicale ou dentaire suivant la revendication 8, dans laquelle la quantité de ce monomère de résine photopolymérisable et photoclivable (P&P) dans un mélange comprenant l'autre résine va de 0,01 % à 99,99 %, avantageusement de 0,5 à 99,5 %, plus avantageusement de 1,0 à 60,0 %, en poids / poids, respectivement.

10. Matière médicale ou dentaire suivant la revendication 8, dans laquelle le système photopolymérisable et photoréactif est également activable par une seconde source d'énergie, séparée de la première source utilisée pour la photopolymérisation de la première réaction, une petite fraction inférieure à 10 % de cette résine pouvant ainsi de préférence être utilisée comme ingrédient fonctionnel pour activer la réaction ou l'événement secondaire sur commande de la manière requise.

11. Matière médicale ou dentaire suivant la revendication 10, dans laquelle une telle commande peut être n'importe quelle énergie extérieure telle que la chaleur, de la lumière à une longueur d'onde donnée, des micro-ondes, une vibration ultrasonique ou une vibration mécanique.

12. Matière médicale ou dentaire suivant les revendications 10 et 11, dans laquelle un tel événement secondaire ou une telle réaction secondaire comprend une modification de viscosité, une régénération d'amine, une régénération d'acide ; ou dans laquelle une telle composition peut être utilisée dans la préparation de matières à viscosité ajustable ; ou dans laquelle une telle composition peut être utilisée dans des matières à constituant à durcissement en deux parties ; ou dans laquelle une telle composition pourrait être utilisée comme système stable à corrosion.

13. Utilisation d'un monomère de résine photopolymérisable et photoclivable suivant l'une quelconque des revendications 1 à 7 pour la réduction de la contrainte de polymérisation d'une composition en pâte contenant une charge.

14. Utilisation suivant la revendication 13, dans laquelle la composition de résine suivant les revendications 1 et 2 est utilisée dans la composition en pâte en une quantité de 1 % à 99 % en poids / poids, de préférence dans laquelle la charge est une composition de charge contenant diverses dimensions, une distribution de dimensions comprenant une nano-charge, une micro-charge, une macro-charge ou une charge hybride en termes de dimensions et de composition ou de nature, qui est inorganique ou organique ou bien prépolymérisée.

15. Utilisation suivant les revendications 13 et 14, dans laquelle la composition de charge est traitée en surface avec des agents de couplage, un silane polymérisable ou non polymérisable ou leur association pour améliorer davantage une propriété physique ou mécanique.
